# EUROPEAN PATENT SPECIFICATION

(11) **EP 1 980 613 B1**
(45) Date of publication and mention of the grant of the patent: **06.01.2016**
(21) Application number: 07706702.3
(22) Date of filing: 12.01.2007
(51) Int. Cl.: C12N 5/071

(54) **METHOD OF MAINTAINING THE FUNCTION OF LIVER TISSUE CELLS OVER LONG TIME**
VERFAHREN ZUR ERHALTUNG DER FUNKTION VON LEBERGEWEBEZELLEN ÜBER EINEN LANGEN ZEITRAUM
PROCEDE DE MAINTIEN DE LA FONCTION DES CELLULES DE TISSUS DU FOIE SUR UNE PERIODE PROLONGEE

(30) Priority: 12.01.2006 JP 2006033057
(43) Date of publication of application: 15.10.2008
(73) Proprietor: TOKYO WOMEN'S MEDICAL UNIVERSITY, Shinjuku-ku Tokyo 162-8666 (JP)
(72) Inventor: YAMATO, Masayuki, Tokyo 1580097 (JP); OHASHI, Kazuo, Osaka 584-0033 (JP); OKANO, Teruo, Chiba 272-0827 (JP)
(74) Representative: Hoffmann Eitle
(86) International application number: PCT/JP2007/050360
(87) International publication number: WO 2007/080990

(56) References cited:
- EP-A- 1 600 177
- YAMADA N, OKANO T, SAKAI H, KARIKUSA F, SAWASAKI Y, SAKURAI Y: "Thermo-responsive polymeric surfaces; control of attachment and detachment of cultured cells" MAKROMOL. CHEM., RAPID COMMUN., vol. 11, no. 11, March 1990 (1990-03), pages 571-576, XP002522610
- OKANO T ET AL: "A NOVEL RECOVERY SYSTEM FOR CULTURED CELLS USING PLASMA-TREATED POLYSTYRENE DISHES GRAFTED WITH POLY(N-ISOPROPYLACRYLAMIDE)" JOURNAL OF BIOMEDICAL MATERIALS RESEARCH, WILEY, NEW YORK, NY, US, vol. 27, no. 10, 1 October 1993 (1993-10-01), pages 1243-1251, XP008020855 ISSN: 0021-9304
- TSUDA Y ET AL: "The use of patterned dual thermoresponsive surfaces for the collective recovery as co-cultured cell sheets" BIOMATERIALS, ELSEVIER SCIENCE PUBLISHERS BV., BARKING, GB, vol. 26, no. 14, 1 May 2005 (2005-05-01), pages 1885-1893, XP025280758 ISSN: 0142-9612 [retrieved on 2005-05-01]
- FUNATSU KAZUMORI ET AL: "Hybrid artificial liver using hepatocyte organoid culture" ARTIFICIAL ORGANS, BLACKWELL SCIENTIFIC PUBLICATIONS, INC., BOSTON, US, vol. 25, no. 3, 1 March 2001 (2001-03-01), pages 194-200, XP002268620 ISSN: 0160-564X
- YAMATO M, OKANO T: "Cell sheet engineering" MATERIALS TODAY, vol. 7, no. 5, April 2004 (2004-04), pages 42-47, XP002522611
- KIKUCHI A ET AL: "TWO-DIMENSIONAL MANIPULATION OF CONFLUENTLY CULTURED VASCULAR ENDOTHELIAL CELLS USING TEMPERATURE-RESPONSIVE POLY(N-ISOPROPYLACRYLAMIDE)-GRAFTED SURFACES" JOURNAL OF BIOMATERIALS SCIENCE. POLYMER EDITION, VSP, UTRECHT, vol. 9, no. 12, 1 January 1998 (1998-01-01), pages 1331-1348, XP002947553 ISSN: 0920-5063
- HARIMOTO M. ET AL.: 'Novel approach for achieving double-layered cell sheets co-culture: overlaying endothelial cell sheets onto monolayer hepatocytes utilizing temperature-responsive culture dishes' J. BIOMED. MATERIALS RES. vol. 62, no. 3, 05 December 2002, pages 464 - 470, XP002995879
- OHASHI K. ET AL.: 'Liver tissue engineering at extrahepatic sites in mice as a potential new therapy for genetic liver diseases' HEPATOLOGY vol. 41, no. 1, January 2005, pages 132 - 140, XP003016091
- YAMATO M. ET AL.: 'Hi Shinshuteki ni Baiyo Saibo o Kaishu dekiru Intelligent Baiyozara: Baiyo Saibo Sheet kara no Soshiki Dukuri' THE JAPANESE JOURNAL OF ARTIFICIAL ORGANS vol. 28, no. 3, 15 August 1999, pages 577 - 581, XP003016092
- SOTO-GUTIERREA A. ET AL.: 'Reversal of mouse hepatic failure using an implanted liver-assist device containing ES cell-derived hepatocytes' NATURE BIOTECH. vol. 21, no. 11, January 2006, pages 1412 - 1419, XP003016093
- KIKUCHI A. ET AL.: 'Two-dimensional manipulation of confluently cultured vascular endothelial cells using temperature-responsive poly(N-isopropylacrylamide)-grafted surfaces' J. BIOMATER. SCI. POLYMER EDN. vol. 9, no. 12, 1998, pages 1331 - 1348, XP002947553

## Description

### TECHNICAL FIELD

The present disclosure relates to a method of maintaining the function of liver tissue cells useful in the fields of biology, medicine, pharmacy and the like for a long period of time and a method for producing liver tissue cells for transplantation which enables long-term maintenance of the function of liver tissue cells to be used in the method.

### BACKGROUND TECHNIQUE

The liver is an central organ of the metabolic function such as glyconeogenesis, storage of glycogen, lipid metabolism, production of plasma proteins, bilirubin metabolism, hormone metabolism and vitamin metabolism, and additionally has complicated functions of performing the production of bile, detoxication by various enzymes and biophylaxis against foreign substances as well. Once a disease such as hepatitis, liver cirrhosis and liver cancers appears in the organ, the living body with the above described damaged function is remarkably adversely affected. And, when the disease proceeds, suitable measures to act for such functions have to be taken.

In such a background, techniques of acting for the function of the liver outside the living body have been considered. And, these techniques mostly act for part of the functions of the liver are merely to filter toxic substances in the blood through a flat membrane or a hollow fiber membrane [for example, Japanese Patent Application No. H06-101775 (Japanese Patent Publication A No. H07-284531) and Japanese Patent Application No. 2004-155864 (Japanese Patent Publication A No. 2004-358343)]. Recently, so as to even slightly act for complicated functions of the liver, the development of hybrid type artificial livers obtained by sealing hepatic cells within the above described filtering device and circulating blood therethrough to act for the function of the liver has been advanced [for example, Japanese

Patent Application No. H08-511686 (Japanese Patent Publication A No. H10-506806) and Japanese Patent Application No. 2002-204967 (Japanese Patent Publication A No. 2004-41527)]. However, the technique herein which performs the function of the liver through extracorporeal circulation of blood was inferior in efficiency and could not necessarily meet the time restriction of possible extracorporeal circulation. Further, there was a problem that the hepatic cells taken outside the living body were inferior in stability and the function of the liver remarkably attenuated in use.

On the other hand, Japanese Patent Publication A No. H05-192138 describes a method of culturing dermal cells comprising culturing dermal cells on a cell culture support where the surface of a base material is coated with a polymer having an upper or lower critical dissolution temperature in water of 0 to 80°C at a temperature below the upper dissolution critical temperature or above the lower critical dissolution temperature and thereafter regulating the culturing temperature to a temperature above the upper limit dissolution temperature or below the lower limit critical dissolution temperature to peel the cultured dermal cells. According to this method, cells are peeled from the culture base material coated with a temperature-responsive polymer but this patent document did not describe a method of producing hepatic tissue cells which maintains the function over a long period time with the use of the cells obtained by this method.

Okano et al. prepared a hepatic parenchymal cell sheet and a vascular endothelial cell sheet with the use of the technique of Japanese Patent Publication A No. H05-192138, laminated the two sheets to bond each other and constructed a co-culture system where hepatic parenchymal cells and vascular endothelial cells interact outside the living body, and as the result, succeeded in the production of albumin, a hepatic plasma protein, as one function of the liver over about 40 days [Journal of Biomedical Material Research, 62, 464-470(2002)]. By this method, hepatic parenchymal cells which easily die out outside the living body were allowed to maintain the cell activity over a long period of time. However, examination of further lengthening the period for maintaining the function was not done, and accordingly the establishment of a novel technique of maintaining the function of hepatic tissue cells for a long period of time which is useful in the fields of biology, medicine, pharmacy and the like has been demanded.

### DISCLOSURE OF THE INVENTION

### PROBLEM TO BE SOLVED BY THE INVENTION

The present invention is made to intend to solve the problems of the above described conventional techniques. In other words, the present invention has an object to provide a hepatic tissue cell sheet for use in medicine and the use of a non-human animal transplanted with a hepatic tissue cell sheet as described herein.

### MEANS TO SOLVE THE PROBLEM

In order to solve the above described problem, the present inventors has made an examination from various angles to carry out research and development. As the result, it has been found that the functions of hepatic tissue cells can be maintained for a long period of time by culturing hepatic tissue cells on a cell culture support whose surface is coated with a polymer which shows a change in the hydration force in the temperature range of 0 to 80°C within the temperature range where the hydration force of the polymer is weak, thereafter changing the temperature of the liquid culture medium to a level at which the hydration force of the polymer becomes strong to peel cultured hepatic tissue cells, and then transplanting the obtained hepatic tissue cells into a definite site *in vivo.* Moreover, it has been found that the hepatic tissue cells transplanted into the living body have the function of an artificial liver. The present invention is completed on the basis of this founding.

It is disclosed a method of maintaining the function of hepatic tissue cells over a long period of time comprising culturing hepatic tissue cells on a cell culture support whose surface is coated with a polymer which shows a change in the hydration force in the temperature range of 0 to 80°C within the temperature range where the hydration force of the polymer is weak, thereafter changing the temperature of the liquid culture medium to a level at which the hydration force of the polymer becomes strong to peel cultured hepatic tissue cells, and then transplanting the obtained hepatic tissue cells into a definite site *in vivo.*

Further, it is disclosed an artificial liver utilizing the method of maintaining the function of hepatic tissue cells over a long period of time.

Still further, it is disclosed an animal other than a human in which hepatic tissue cells have been transplanted utilizing the method of maintaining the function of hepatic tissue cells over a long period of time.

Furthermore, it is disclosed a system of evaluating the function of the liver comprising administering a test substance to an animal transplanted with hepatic tissue cells and judging the influence of the test substance on the function of the liver.

Additionally, it is disclosed a method for producing hepatic tissue cells for transplantation which enables long-term maintenance of the function of hepatic tissue cells comprising culturing liver tissue cells on a cell culture support whose surface is coated with a polymer which shows a change in the hydration force in the temperature range of 0 to 80°C within the temperature range where the hydration force of the polymer is weak and thereafter changing the temperature of the liquid culture medium to a level at which the hydration force of the polymer becomes strong to peel the cultured hepatic tissue cells.

Specifically, the present invention provides:
1. A hepatic tissue cell sheet comprising at least hepatic parenchymal cells obtained from hepatic tissue for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or assistance to the function of the liver, which sheet is obtainable by culturing hepatic tissue cells comprising at least hepatic parenchymal cell obtained from hepatic tissue on a cell culture support whose surface is coated with a polymer which shows a change in the hydration force in the temperature range of 0 to 80°C within the temperature range where the hydration force of the polymer is weak, thereafter changing the temperature of the liquid culture medium to a level at which the hydration force of the polymer becomes strong to peel cultured hepatic tissue cells, that are suitable for transplantation into a definite site in the living body of an animal including a human.
2. The hepatic tissue cell sheet according to item 1 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or assistance to the function of the liver, wherein the function of the liver can be varied by changing the size and/or the shape of the sheet.
3. The hepatic tissue cell sheet according to item 1 or 2 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or assistance to the function of the liver, characterized in that said sheet produces at least one of human blood coagulation factor VIII, human blood coagulation factor IX, human alpha-1 antitrypsin and albumin.
4. The hepatic tissue cell sheet according to item 3 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or assistance to the function of the liver, wherein in the treatment of haemophilia an effective gene for the production of human blood coagulation factor VIII and/or human blood coagulation factor IX is introduced into the hepatic tissue cell sheet.
5. The hepatic tissue cell sheet according to any one of items 1 to 4 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or assistance to the function of the liver, wherein the site to be transplanted is subcutaneous tissue, intraperitoneal tissue, the liver or a muscle within the living body.
6. The hepatic tissue cell sheet according to any one of items 1 to 5 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or assistance to the function of the liver, wherein the site to be transplanted is previously provided with a treatment of the induction of angiogenesis.
7. The hepatic tissue cell sheet according to item 6 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or assistance to the function of the liver, wherein the method of inducing angiogenesis is performed by the FGF treatment.
8. Use of a non-human animal transplanted with a hepatic tissue cell sheet as defined in any one of items 1 to 7 for evaluating the function of the liver comprising judging the function of the liver under the influence of a test substance after said test substance has been administered to the non-human animal.
9. Use according to item 8, wherein the animal is a rat, a mouse, a guinea pig, a marmoset, a rabbit, a dog, a pig, a chimpanzee or an immunodeficient animal thereof.

### EFFECT OF THE INVENTION

The method of maintaining the function of hepatic tissue cells for a long period of time described herein would enable long-term maintenance of the function of cultured hepatic tissue cells, would enable carrying out long-term fundamental research relating to the function of the liver as a whole, and furthermore would enable production of artificial livers and animals transplanted with hepatic tissue cells which require the expression of the function of the live over a long period of time.

### BRIEF EXPLANATION OF THE DRAWINGS

Fig. 1 is photographs showing a section of the transplanted tissue 120 days after transplantation in Example 1.
Fig. 2 is photographs showing sections of the transplanted tissue 120 days after transplantation in Example 1.
Fig. 3 is a series of graphs showing the result of measuring the serum hAAT concentrations in Example 1.
Fig. 4 is a series of graphs showing the amount of human alpha-1 antitrypsin (hA1AT) produced, the amount of albumin produced and the amount of lidocaine metabolized by the hepatic tissue cell sheet of the present invention and by the individual cells obtained by the conventional method in Example 2.

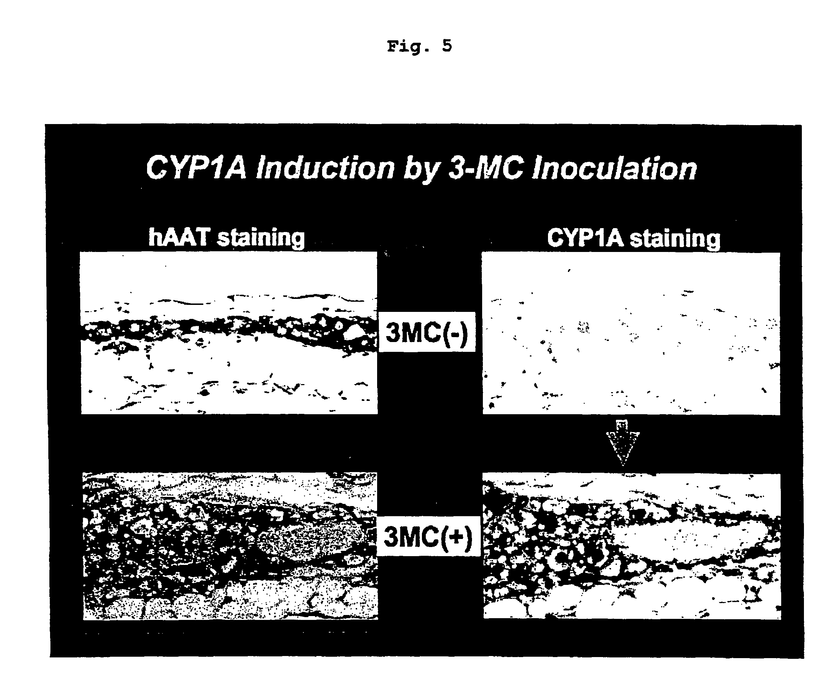

Fig. 5 is a series of tissue staining views showing the induction of CYP1A of a drug metabolizing enzyme with or without inoculation of 3-MC (3-methylcholantrene) in Example 4.
Fig. 6 is a graph showing the results of measuring the serum hAAT levels in Example 4.

### BEST MODE FOR CARRYING OUT THE INVENTION

The hepatic tissue cells which are used in the present invention are cells collected from the hepatic tissue which may contain hepatic parenchymal cells to express the function of the liver. The other cells may be either nonparenchymal cells within the hepatic tissue or other cells, and include, for example, sinusoidal endothelial cells, Kupffer cells, stellate cells, pit cells, bile duct epithelial cells, vascular endothelial cells, fibroblasts and mesenchymal stem cells, and are not particularly limited. Further, these cells include cells directly collected from tissue and cell lines and their types are not limited. The origin of these cells is not particularly limited and includes, for example, a rat, a mouse, a guinea pig, a marmoset, a rabbit, a dog, a cat, a sheep, a pig, a chimpanzee and an immunodeficient animal thereof, and in the case of using liver tissue cells in the treatment of humans, cells originated from a pig and a chimpanzee are desirably used. The medium for cell culturing in the present invention is not particularly limited if the medium is the one conventionally used for the cells to be cultured.

The number of cells seeded on culturing in the present invention varies depending on the animal species of the cells used and is typically not less than 1 x 10⁴/cm², preferably not less than 3 x 10⁴/cm² and more preferably, not less than 6 x 10⁴/cm². With seeding concentrations of less than 1 x 10⁴/cm², the proliferation of hepatic tissue cells is inferior to deteriorate the extent of expression of the function of the obtained hepatic tissue cells and such concentrations are not preferred in the present invention.

In the present invention, the above described cells are cultured on a cell culture support whose surface is coated with a polymer which changes in the hydration force in the temperature range of 0 to 80°C in the temperature range where the hydration force of the polymer is weak. Normally, the temperature for culturing the cells is preferably 37°C. The temperature-responsive polymer which is used in the present invention may be either a homopolymer or a copolymer. Such a polymer includes, for example, polymers described in Japanese Patent Publication A No. H02-211865. Specifically, for example, the polymer can be obtained by homopolymerization or copolymerization of the following monomers. The monomers which can be used include, for example, a (meth)acrylamide compound, an N-(or N,N-di)alkyl substituted (meth)acrylamide derivative and a vinyl ether derivative and in the case of a copolymer, any two or more kinds of these compounds can be used. Furthermore, copolymers with monomers other than the above described monomers, graft polymers or copolymers of the polymers with one another and mixtures of the polymers with the copolymers may be used. Further, it is possible to crosslink the polymer within the range of not damaging the inherent properties of the polymer. The method of coating various kinds of polymers on the surface of a base material is not particularly limited and may follow the method described in Japanese Patent Publication A No. H02-211865. Specifically, such coating can be performed by subjecting the base material and the above described monomer or polymer to any one of electron beam (EB) irradiation, γ-ray irradiation, ultraviolet ray irradiation, plasma treatment, corona treatment and organic polymerization reaction or by physical adsorption such as coating and kneading. The amount of the hydrophilic polymer immobilized at the cell adhering site may be an amount enough to move the cells and is not particularly limited, and since the cells to be used are hepatic tissue cells, their immobilization amount is not less than 0.4 µg/cm², preferably not less than 0.8 µg/cm² and more preferably, not less than 1.0 µg/cm². The amount of the polymer immobilized may be measured in accordance with the conventional method which includes, for example, a method of directly measuring the cell adhered site with the use of FT-IR-ATR and a method of immobilizing a previously labeled polymer in the same manner as above described and estimating the amount of the polymer immobilized from the amount of the labeled polymer immobilized at the cell adhering site, and any method may be used.

The shape of the culture base material in the present invention is not particularly limited and includes, for example, a form of a dish, a multi-plate, a flask or a cell insert and a flat membrane form. As the base material provided with coating, substances capable of generally giving a form such as a polymer compound and a ceramic, including glass, modified glass, a compound such as polystyrene and polymethyl methacrylate which are conventionally used in cell culturing all can be used.

In the cell culture support of the present invention, the temperature-responsive polymer coated on the base material causes hydration and dehydration by changing temperature and the temperature range is found to be 0°C to 80°C, preferably 10°C to 50°C and more preferably, 20°C to 45°C. At temperatures of exceeding 80°C, there is a possibility that the hepatic tissue cells die out, and thus such temperatures are not preferred. Further, at temperatures lower than 0°C, in general, the proliferation rate of cells is extremely decreased or the hepatic tissue cells die out, and such temperatures are not preferred, either.

When the cultured cells are peeled and recovered from the support material in the method of the present invention, the cultured hepatic tissue cells are allowed to adhere to a carrier, if necessary, and can be peeled as they are together with the carrier by setting the temperature of the support base material to which the cells adhere at a temperature of the hydration of the coated polymer of the support base material. In this instance, a water flow may be applied in between the cell sheet and the support to smoothly perform peeling. Further, peeling of the sheet may be performed in the liquid culture medium where the cells have been cultured or in other isotonic solutions, and the peeling method can be selected in accordance with the object.

The cultured hepatic tissue cells in the present invention are not susceptible to the damage by a proteolytic enzyme represented by dispase, trypsin or the like on culturing. Therefore, the hepatic tissue cells peeled from the base material have an adherent protein and when the hepatic tissue cells are peeled in the form of a sheet, the cell-to-cell desmosome structure is to be maintained to some extent. On account of this, the hepatic tissue cell sheet can well adhere to the tissue of an affected part on transplantation and transplantation can be efficiently performed. It is known that in general, with respect to dispase of a proteolytic enzyme, the cell-to-cell desmosome structure can be peeled while maintaining 10 to 40% thereof but a cell-to-base material basement membrane-like protein or the like is mostly destructed and accordingly, the strength of the obtained cell sheets becomes weak. In contrast to this, the hepatic tissue cell sheet of the present invention is in the state that not less than 60% of both the desmosome structure and the basement membrane-like protein remain, and various effects as described above can be obtained.

When the hepatic tissue cells in the present invention are in the form of a sheet, the sheet may be a laminate of a plurality of sheets and the number of lamination is not particularly limited. Lamination of the hepatic tissue cell sheet improves the cell density per sheet unit area to improve the function as a hepatic tissue cell sheet, and accordingly is preferred.

The above will be explained by taking poly(N-isopropylacrylamide) as an example. It is known that poly(N-iso-propylacrylamide) has a lower limit dissolution temperature at 31°C and causes dehydration in water at a temperature of not lower than 31°C if it is in a free state and the polymer chain is coagulated to become cloudy. Inversely, at temperatures of not higher than 31°C, the polymer chain causes hydration to become in a state dissolved in water. In the present invention this polymer is coated and immobilized on the surface of a base material such as a petri dish. Accordingly, at temperatures of not lower than 31°C, the polymer on the surface of the base material causes dehydration in the same manner but the polymer chain is coated and immobilized on to the surface of the base material, and thus the surface of the base material shows hydrophobicity. Inversely, at temperatures of not higher than 31°C, the polymer on the surface of the base material causes hydration but the polymer chain is coated and immobilized on the surface of the base material, and thus the surface of the base material is to show hydrophilicity. The hydrophobic surface at this time is a suitable surface to which cells can adhere to proliferate, and further the hydrophilic surface becomes a surface to which the cells cannot adhere and the cells or the cell sheet during culturing is to be peeled only by cooling.

The carrier which is used in allowing hepatic tissue cells or a hepatic tissue cell sheet to adhere thereto is a structure for holding the cells of the present invention and, for example, a polymeric membrane or a structure molded from the polymeric membrane, a metallic jig or the like can be used as the carrier. For example, in the case of using a polymer as the material of the carrier, specific materials include, for example, polyvinylidene difluoride (PVDF), polypropylene, polyethylene, cellulose and its derivative, paper, chitin, chitosan, collagen and urethanes. The shape of the carrier is not particularly limited.

In the present invention, the obtained hepatic tissue cells or hepatic tissue cell sheet is to be transplanted into a predetermined site within the living body. The site to be transplanted may be any site within the living body and is not particularly limited. The site to be transplanted includes, for example, subcutaneous tissue, intraperitoneal tissue, the liver, and a muscle. The site to be transplanted may or may not be previously provided with the induction of angiogenesis and is not particularly limited. Herein, the method of inducing angiogenesis is not particularly limited and includes, for example, a method of embedding FGF (fibroblast growth factor) of a blood growth factor in a microsphere and allowing the microsphere to act within the living body for 8 to 10 days while changing the composition, the size and the range of injection of this microsphere, a method of cutting a polyethylene terephthalate mesh to any size to prepare a bag, placing a high concentration agarose solution dissolving FGF in the bag and thereafter eliminating the bag after 8 to 10 days to prepare an angiogenesis induced space

The hepatic tissue cells to be transplanted in the present invention maintain the basement membrane-like protein and have extremely good bioadhesiveness. The number of cells may vary in transplantation depending on the object, and when the cells to be transplanted are in the form of a sheet, the total activity of the function of the liver can be varied by changing the size or the shape of the sheet.

When the method of maintaining hepatic tissue cells for a long period of time of the present invention is utilized for humans, the transplanted hepatic tissue cells or hepatic tissue cell sheet is to express the function of the liver in the human living body for a long period of time to come to an artificial liver as it is. When the peeled hepatic tissue cells are in the form of a sheet, the amount of expression of the function can be controlled by the size or the shape of the sheet or both. Such an artificial liver is used with the object of the substantial treatment of each disease illustrated by hepatic enzyme deficiency, hemophilia, coagulopathy, liver failure, fulminant hepatitis, chronic hepatitis, liver cirrhosis, a liver resected patient and an infectious disease or assistance to the function of the liver, and its use is not particularly limited. When hemophilia is explained here, the deficiency of human blood coagulation factor VIII and/or human blood coagulation factor IX is the cause of the substantial onset of hemophilia, and the artificial liver of the present invention is to produce these factors to supplement them. In this instance, in order to increase their production capacity, for example, an effective gene for the production of human blood coagulation factor VIII and/or human blood coagulation factor IX may be introduced into the hepatic tissue cell sheet of the present invention as the conventional technique, and its method is not particularly limited.

When the method of maintaining hepatic tissue cells for a long period of time of the present invention is utilized for animals, the transplanted hepatic tissue cells or hepatic tissue sheet is to express the function of the liver within the living bodies of the animals, in other words, to come to animals transplanted with hepatic tissue cells as it is. When the peeled hepatic tissue cells are in the form of a sheet, the quantity of expression of the function can be controlled by the size or the shape of the sheet or both. The animals which are used herein include, for example, a rat, a mouse, a guinea pig, a marmoset, a rabbit, a dog, a pig, a chimpanzee and an immunodeficient animal thereof, and are not particularly limited. Such animals transplanted with hepatic tissue cells are, for example, used with the object of the system of evaluating the function of the liver by administering a test substance to this animal transplanted with hepatic tissue cells to judge the influence of the test substance on the function of the liver or the like but their use is not particularly limited thereto.

### Examples

The present invention will now be explained in detail on the basis of examples which by no means limit the present invention.

### [Example 1]

On a cell culture base material, poly(N-isopropylacrylamide) of a temperature-responsive polymer was coated to 2.0 µg/cm², and hepatic tissue cells (number of seeding cells: 2 x 10⁴, 37°C, 5% CO₂) were cultured. After three days it was confirmed that the hepatic tissue cells on the cell culture base material became confluent, and thereafter parchment paper (SS-25) was selected as a carrier for moving the cultured cells and left to stand on the cultured cell sheet. The cultured hepatic tissue cells were allowed to adhere on to the SS-25 and the cell culture base material was cooled at 20°C for 60 minutes. After cooling, the peeled cell sheet was collected from the carrier and was subcutaneously transplanted into a mouse in accordance with the method of transplanting hepatic tissue cells as shown below.

### (Method of Transplanting Hepatic Cell Sheet)

(1) A mouse is put under general anesthesia. A sustained release device obtained by dissolving bFGF in agarose is subcutaneously inserted into the mouse and subjected to the induction of angiogenesis in ten days.
(2) Hepatic cells are separated and cultured for three days to bring about a confluent state.
(3) The circumference of the confluent hepatic cells is peeled with the tip of a pipette.
(4) After about 20 minutes (which vary dependent on the lot of the dish), the SS-25 which has been cut to the size slightly larger than the size of the hepatic cell sheet and has slightly been moistened with the medium is placed on the hepatic cell sheet and left to stand for 30 seconds.
(5) Only part of the hepatic cell sheet on the external side of the SS-25 is turned up and peeling of the hepatic cell sheet is carefully started therefrom.
(6) The mouse is put under general anesthesia. The device is removed and the hepatic cells peeled with the SS-25 attached is placed into the subcutaneous space of the mouse having undergone the induction of angiogenesis, and after waiting for about 20 seconds, peeling of the SS-25 from the hepatic cell sheet is carefully started.
(7) The wound is closed.

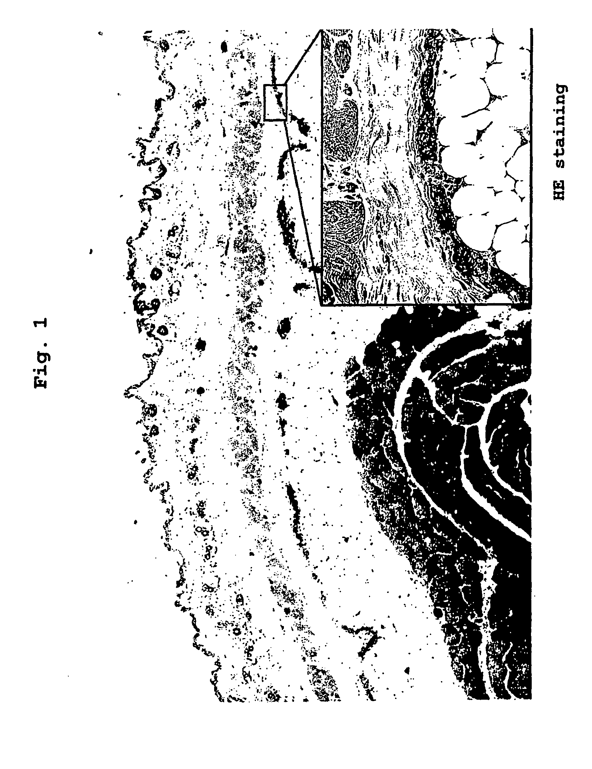

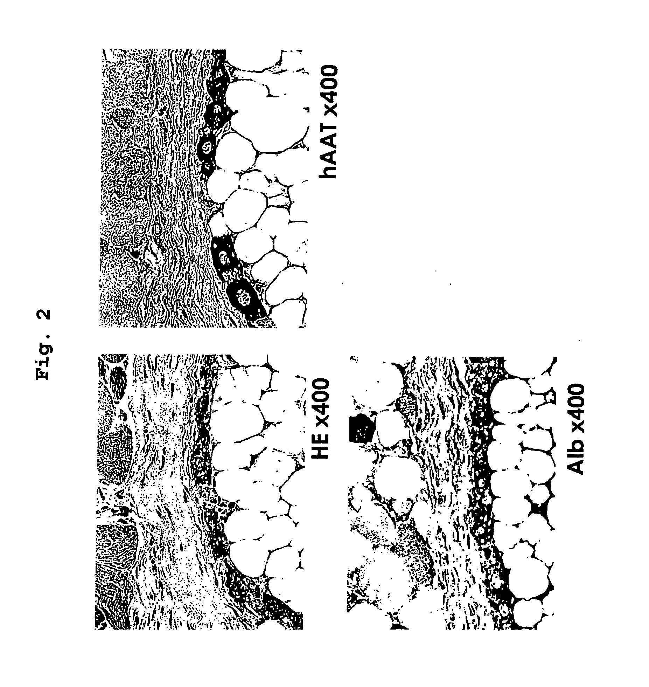

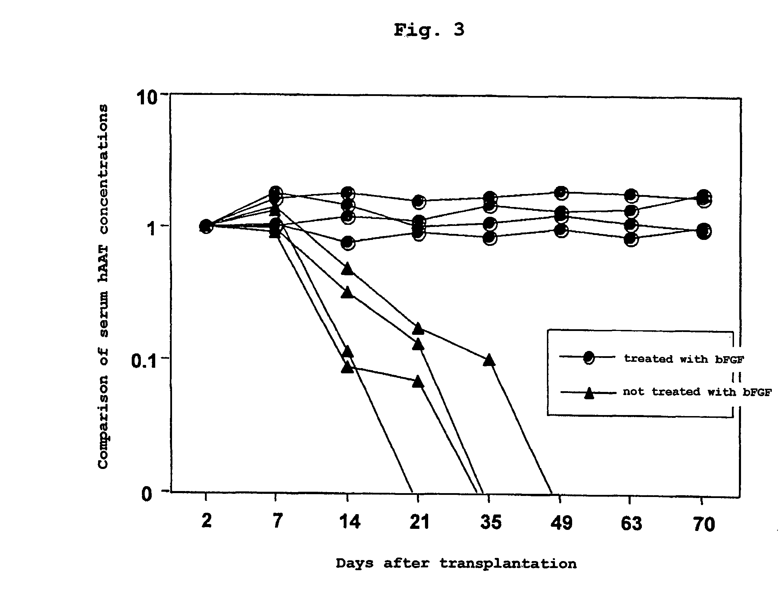

The section of the tissue of the transplanted part 120 days after transplantation is shown in Fig. 1 and Fig. 2. The enlarged photograph in Fig. 1 is an enlargement of the inside of a rectangle in the Figure. Further, the part stained with HE, the part staining hAAT and the part staining albumin are shown in Fig. 2, respectively. The function of the liver was confirmed by measuring the murine serum hAAT concentrations in accordance with the conventional method. The obtained results are shown in Fig. 3. It can be understood that according to the technique of the present invention, the hepatic tissue cell sheet transplanted into the site having undergone the induction of angiogenesis with bFGF expresses the function of the liver over a long period opt time.

### [Comparative Example 1]

The same experiment as in Example 1 was carried out except that the sustained release device obtained by dissolving bFGF in agarose was not subcutaneously inserted into the mouse and the mouse did not undergo the induction of angiogenesis. The obtained results are shown in Fig. 3.

### [Example 2 and Comparative Example 2]

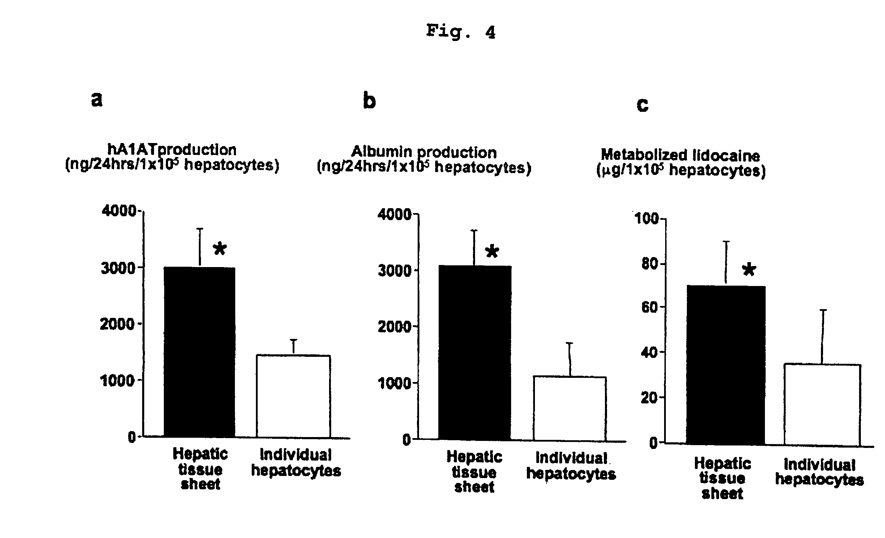

On a cell culture base material, poly(N-isopropylacrylamide) of a temperature-responsive polymer was coated to 1.9 µg/cm², and hepatic tissue cells (number of seeding cells: 2 x 10⁴, 37°C, 5% CO₂) were cultured. Three days after culturing, the temperature of the culture base material was set at 20°C and cooled for 15 minutes to recover a hepatic tissue cell sheet. The hepatic cell sheet was applied to a new culture plate and was recultured to prepare a hepatic tissue sheet group (Fig. 4a) (Example 2). Further, by treating the cells cultured under the same conditions with collagenase of a proteolytic enzyme at the same time to individually divide the cells, the cells were recovered in a divided state, and thereafter recultured on a new culture plate to prepare an individual hepatocyte group (Comparative Example 2). The amount of human alpha-1 antitrypsin (Fig. 4c) and the amount of albumin (Fig. 4b) which were produced in the culture medium 24 hours after starting re-culturing were evaluated by comparing both groups. As to Fig. 4c, 1 mg of lidocaine was added to the culture medium 24 hours after starting re-culturing and the amount of lidocaine remaining in the culture medium four hours after addition of lidocaine was measured and evaluation was made by measuring the amount of the lidocaine metabolized by the cultured cells of both groups. Respective values are given on the basis of the number of the re-cultured cells of 1 x 10⁵. In all evaluations of a to c, the hepatic tissue sheet group obtained a significantly (P<0.01) good result. It is understood that according to the technique of the present invention, the hepatic tissue cell sheet expresses the function of the liver over a long period of time.

### [Example 3 and Comparative Example 3]

The murine hepatic tissue cell sheet as obtained in Example 1 was transplanted by application into a murine subcutaneous site having undergone the induction of angiogenesis in accordance with the method of transplanting a hepatic cell sheet as shown in Example 1. One hundred twenty days after transplantation, 3-methylcholantrene (Sigma-Aldrich, St. Louis, MO) was intraperitoneally administered once a day and continuously for three days, and 24 hours after the final administration, the mouse was killed and the hepatic tissue prepared was subjected to tissue staining (Example 3). With the prepared murine subcutaneous hepatic tissue (Comparative Example 3) without being administered with 3-methylcholantrene, CYP1A of a drug metabolizing enzyme was not induced while with the prepared murine subcutaneous hepatic tissue administered with 3-methylcholantrene, CYP1A was strongly induced. It can be understood that according to the technique of the present invention, the hepatic tissue cell sheet expresses the function of the liver over a long period of time. Further, CYP1A means CYP1A activity.

### [Example 4]

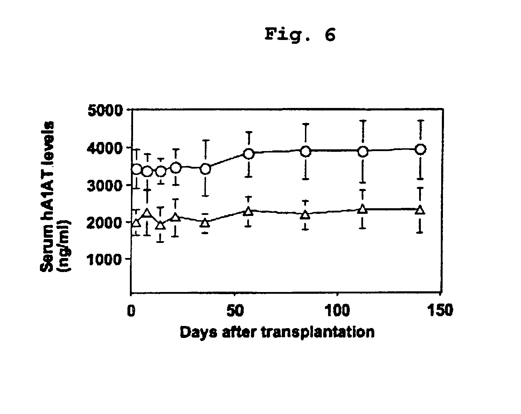

A larger hepatic tissue sheet was prepared in accordance with the method as shown in Example 1 by laminating the murine hepatic tissue cell sheets on transplantation. Specifically, a group (mark Δ in Fig. 6) obtained by transplanting one murine hepatic tissue sheet into the murine subcutaneous site having undergone the induction of angiogenesis and a group (mark o in Fig. 6) obtained by transplanting the two sheets thereinto were prepared. With the two groups, the blood human alpha-1 antitrypsin concentration of the transplanted mice was measured up to 140 days after transplantation and evaluation was made by examining the stability of the transplanted tissue and the change of the amount of the tissue. It has been found that with the group transplanted with the two hepatic tissue cell sheets, a significantly large tissue was prepared compared with the group transplanted with the one sheet, and furthermore the tissue stably exists.

### INDUSTRIAL UTILITY

Utilization of the method of maintaining the function of hepatic tissue cells over a long time as shown herein would enable long-term maintenance of the function of cultured hepatic tissue cells, would enable long-term fundamental research relating to the function of the liver and also would enable production of artificial livers and hepatic tissue cell transplanted animals which require the expression of the function of the liver over a long period of time.

## Claims

1. A hepatic tissue cell sheet comprising at least hepatic parenchymal cells obtained from hepatic tissue for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or for use in assistance to the function of the liver, which sheet is obtained by culturing hepatic tissue cells comprising at least hepatic parenchymal cell obtained from hepatic tissue on a cell culture support whose surface is coated with a polymer which shows a change in the hydration force in the temperature range of 0 to 80°C within the temperature range where the hydration force of the polymer is weak, thereafter changing the temperature of the liquid culture medium to a level at which the hydration force of the polymer becomes strong to peel cultured hepatic tissue cells, that are suitable for transplantation into a definite site in the living body of an animal including a human.

2. The hepatic tissue cell sheet according to claim 1 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or for use in assistance to the function of the liver, wherein the function of the liver can be varied by changing the size and/or the shape of the sheet.

3. The hepatic tissue cell sheet according to claim 1 or 2 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or for use in assistance to the function of the liver, **characterized in that** said sheet produces at least one of human blood coagulation factor VIII, human blood coagulation factor IX, human alpha-1 antitrypsin and albumin.

4. The hepatic tissue cell sheet according to claim 3 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or for use in assistance to the function of the liver, wherein in the treatment of haemophilia an effective gene for the production of human blood coagulation factor VIII and/or human blood coagulation factor IX is introduced into the hepatic tissue cell sheet.

5. The hepatic tissue cell sheet according to any one of claims 1 to 4 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or for use in assistance to the function of the liver, wherein the site to be transplanted is subcutaneous tissue, intraperitoneal tissue, the liver or a muscle within the living body.

6. The hepatic tissue cell sheet according to any one of claims 1 to 5 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or for use in assistance to the function of the liver, wherein the site to be transplanted is previously provided with a treatment of the induction of angiogenesis.

7. The hepatic tissue cell sheet according to claim 6 for use in the treatment of each disease illustrated by hepatic enzyme deficiency, haemophilia, coagulopathy, liver failure, fulminant hepatitis, chronic hepatitis, liver cirrhosis, a liver resected patient and an infectious disease, or for use in assistance to the function of the liver, wherein the method of inducing angiogenesis is performed by the FGF treatment.

8. Use of a non-human animal transplanted with a hepatic tissue cell sheet as defined in any one of claims 1 to 7 for evaluating the function of the liver comprising judging the function of the liver under the influence of a test substance after said test substance has been administered to the non-human animal.

9. Use according to claim 8, wherein the animal is a rat, a mouse, a guinea pig, a marmoset, a rabbit, a dog, a pig, a chimpanzee or an immunodeficient animal thereof.

## Patentansprüche

1. Lebergewebe-Zellschicht, umfassend wenigstens aus Lebergewebe gewonnene parenchymale Leberzellen, zur Verwendung bei der Behandlung jeder Krankheit, die durch Leberenzymdefizit zum Vorschein kommt, von Hämophilie, Koagulopathie, Leberversagen, fulminanter Hepatitis, chronischer Hepatitis, Leberzirrhose, eines Patienten mit resezierter Leber und einer Infektionserkrankung, oder zur Verwendung bei der Unterstützung der Leberfunktion, wobei die Schicht gewonnen wird durch Kultivieren von Lebergewebe-Zellen, die zumindest aus Lebergewebe gewonnene parenchymale Leberzellen umfassen, auf einem Zellkulturträger, dessen Oberfläche mit einem Polymer beschichtet ist, das eine Änderung der Hydratationskraft im Temperaturbereich von 0 bis 80°C zeigt, innerhalb des Temperaturbereichs, in dem die Hydratationskraft des Polymers schwach ist, und dann Änderung der Temperatur des flüssigen Kulturmediums bis zu einem Niveau, bei dem die Hydratationskraft des Polymers ausreichend stark wird, um kultivierte Lebergewebe-Zellen abzuziehen, die zur Transplantation an eine bestimmte Stelle des lebenden Körpers eines Tieres, einschließlich eines Menschen, geeignet sind.

2. Lebergewebe-Zellschicht gemäß Anspruch 1 zur Verwendung bei der Behandlung jeder Krankheit, die durch Leberenzymdefizit zum Vorschein kommt, von Hämophilie, Koagulopathie, Leberversagen, fulminanter Hepatitis, chronischer Hepatitis, Leberzirrhose, eines Patienten mit resezierter Leber und einer Infektionserkrankung, oder zur Verwendung bei der Unterstützung der Leberfunktion, wobei die Leberfunktion durch Änderung der Größe und/oder der Form der Schicht variiert werden kann.

3. Lebergewebe-Zellschicht gemäß Anspruch 1 oder 2 zur Verwendung bei der Behandlung jeder Krankheit, die durch Leberenzymdefizit zum Vorschein kommt, von Hämophilie, Koagulopathie, Leberversagen, fulminanter Hepatitis, chronischer Hepatitis, Leberzirrhose, eines Patienten mit resezierter Leber und einer Infektionserkrankung, oder zur Verwendung bei der Unterstützung der Leberfunktion, **dadurch gekennzeichnet, dass** die Schicht wenigstens eines aus humanem Blutkoagulationsfaktor VIII, humanem Blutkoagulationsfaktor IX, humanem Alpha-1-Antitrypsin und Albumin produziert.

4. Lebergewebe-Zellschicht gemäß Anspruch 3 zur Verwendung bei der Behandlung jeder Krankheit, die durch Leberenzymdefizit zum Vorschein kommt, von Hämophilie, Koagulopathie, Leberversagen, fulminanter Hepatitis, chronischer Hepatitis, Leberzirrhose, eines Patienten mit resezierter Leber und einer Infektionserkrankung, oder zur Verwendung bei der Unterstützung der Leberfunktion, wobei bei der Behandlung von Hämophilie ein effektives Gen zur Produktion von humanem Blutkoagulationsfaktor VIII und/oder humanem Blutkoagulationsfaktor IX in die Lebergewebe-Zellschicht eingebracht wird.

5. Lebergewebe-Zellschicht gemäß einem der Ansprüche 1 bis 4 zur Verwendung bei der Behandlung jeder Krankheit, die durch Leberenzymdefizit zum Vorschein kommt, von Hämophilie, Koagulopathie, Leberversagen, fulminanter Hepatitis, chronischer Hepatitis, Leberzirrhose, eines Patienten mit resezierter Leber und einer Infektionserkrankung, oder zur Verwendung bei der Unterstützung der Leberfunktion, wobei die Transplantationsstelle subkutanes Gewebe, intraperitoneales Gewebe, die Leber oder ein Muskel innerhalb des lebenden Körpers ist.

6. Lebergewebe-Zellschicht gemäß einem der Ansprüche 1 bis 5 zur Verwendung bei der Behandlung jeder Krankheit, die durch Leberenzymdefizit zum Vorschein kommt, von Hämophilie, Koagulopathie, Leberversagen, fulminanter Hepatitis, chronischer Hepatitis, Leberzirrhose, eines Patienten mit resezierter Leber und einer Infektionserkrankung, oder zur Verwendung bei der Unterstützung der Leberfunktion, wobei im Vorfeld an der Transplantationsstelle eine Behandlung zur Induktion von Angiogenese bereitgestellt wird.

7. Lebergewebe-Zellschicht gemäß Anspruch 6 zur Verwendung bei der Behandlung jeder Krankheit, die durch Leberenzymdefizit zum Vorschein kommt, von Hämophilie, Koagulopathie, Leberversagen, fulminanter Hepatitis, chronischer Hepatitis, Leberzirrhose, eines Patienten mit resezierter Leber und einer Infektionserkrankung, oder zur Verwendung bei der Unterstützung der Leberfunktion, wobei das Verfahren zur Induktion von Angiogenese mittels der FGF-Behandlung durchgeführt wird.

8. Verwendung eines nicht-humanen Tieres, das mit einer Lebergewebe-Zellschicht, definiert wie in einem der Ansprüche 1 bis 7, transplantiert ist, zur Evaluierung der Lebefunktion, umfassend Beurteilung der Leberfunktion unter dem Einfluss einer Testsubstanz, nachdem diese Testsubstanz an das nicht-humane Tier verabreicht wurde.

9. Verwendung gemäß Anspruch 8, wobei das Tier eine Ratte, eine Maus, ein Meerschweinchen, eine Marmosette, ein Kaninchen, ein Hund, ein Schwein, ein Schimpanse oder ein immundefizientes Tier davon ist.

## Revendications

1. Feuillet cellulaire de tissu hépatique comprenant au moins des cellules parenchymateuses hépatiques obtenues à partir de tissu hépatique pour une utilisation dans le traitement de chaque maladie illustrée par une déficience en enzymes hépatiques, l'hémophilie, une coagulopathie, une insuffisance hépatique, une hépatite fulminante, une hépatite chronique, une cirrhose hépatique, un patient dont le foie a été reséqué et une maladie infectieuse, ou pour une utilisation dans l'assistance à la fonction du foie, lequel feuillet est obtenu par culture de cellules de tissu hépatique comprenant au moins des cellules parenchymateuses hépatiques obtenues à partir de tissu hépatique sur un support de culture cellulaire dont la surface est revêtue d'un polymère qui présente une variation de la force d'hydratation dans la plage de température de 0 à 80 °C au sein de la plage de températures où la force d'hydratation du polymère est faible, puis le changement de la température du milieu de culture liquide à un niveau auquel la force d'hydratation du polymère devient forte pour décoller les cellules de tissu hépatique cultivées, qui sont appropriées pour une transplantation dans un site défini dans le corps vivant d'un animal y compris un être humain.

2. Feuillet cellulaire de tissu hépatique selon la revendication 1 pour une utilisation dans le traitement de chaque maladie illustrée par une déficience en enzymes hépatiques, l'hémophilie, une coagulopathie, une insuffisance hépatique, une hépatite fulminante, une hépatite chronique, une cirrhose hépatique, un patient dont le foie a été reséqué et une maladie infectieuse, ou pour une utilisation dans l'assistance à la fonction du foie, où la fonction du foie peut varier en changeant la taille et/ou la forme du feuillet.

3. Feuillet cellulaire de tissu hépatique selon la revendication 1 ou 2 pour une utilisation dans le traitement de chaque maladie illustrée par une déficience en enzymes hépatiques, l'hémophilie, une coagulopathie, une insuffisance hépatique, une hépatite fulminante, une hépatite chronique, une cirrhose hépatique, un patient dont le foie a été reséqué et une maladie infectieuse, ou pour une utilisation dans l'assistance à la fonction du foie, **caractérisé en ce que** ledit feuillet produit au moins l'un du facteur de coagulation VIII du sang humain, du facteur de coagulation IX du sang humain, de l'alpha-1-antitrypsine humaine et de l'albumine.

4. Feuillet cellulaire de tissu hépatique selon la revendication 3 pour une utilisation dans le traitement de chaque maladie illustrée par une déficience en enzymes hépatiques, l'hémophilie, une coagulopathie, une insuffisance hépatique, une hépatite fulminante, une hépatite chronique, une cirrhose hépatique, un patient dont le foie a été reséqué et une maladie infectieuse, ou pour une utilisation dans l'assistance à la fonction du foie, où dans le traitement de l'hémophilie un gène efficace pour la production du facteur de coagulation VIII du sang humain et/ou du facteur de coagulation IX du sang humain est introduit dans le feuillet cellulaire de tissu hépatique.

5. Feuillet cellulaire hépatique selon l'une quelconque des revendications 1 à 4 pour une utilisation dans le traitement de chaque maladie illustrée par une déficience en enzymes hépatiques, l'hémophilie, une coagulopathie, une insuffisance hépatique, une hépatite fulminante, une hépatite chronique, une cirrhose hépatique, un patient dont le foie a été reséqué et une maladie infectieuse, ou pour une utilisation dans l'assistance à la fonction du foie, où le site à transplanter est du tissu sous-cutané, du tissu intrapéritonéal, le foie ou un muscle au sein du corps vivant.

6. Feuillet cellulaire de tissu hépatique selon l'une quelconque des revendications 1 à 5 pour une utilisation dans le traitement de chaque maladie illustrée par une déficience en enzymes hépatiques, l'hémophilie, une coagulopathie, une insuffisance hépatique, une hépatite fulminante, une hépatite chronique, une cirrhose hépatique, un patient dont le foie a été reséqué et une maladie infectieuse, ou pour une utilisation dans l'assistance à la fonction du foie, où le site à transplanter est fourni au préalable avec un traitement de l'induction de l'angiogenèse.

7. Feuillet cellulaire de tissu hépatique selon la revendication 6 pour une utilisation dans le traitement de chaque maladie illustrée par une déficience en enzymes hépatiques, l'hémophilie, une coagulopathie, une insuffisance hépatique, une hépatite fulminante, une hépatite chronique, une cirrhose hépatique, un patient dont le foie a été reséqué et une maladie infectieuse, ou pour une utilisation dans l'assistance à la fonction du foie, où le procédé d'induction de l'angiogenèse est effectué par le traitement au FGF.

8. Utilisation d'un animal non humain transplanté avec un feuillet cellulaire de tissu hépatique tel que défini dans l'une quelconque des revendications 1 à 7 pour l'évaluation de la fonction du foie comprenant le jugement de la fonction du foie sous l'influence d'une substance à tester après que ladite substance à tester a été administrée à l'animal non humain.

9. Utilisation selon la revendication 8, où l'animal est un rat, une souris, un cobaye, un marmouset, un lapin, un chien, un porc, un chimpanzé ou un animal immunodéficient parmi ceux-ci.
